Europäisches Patentamt

European Patent Office

Office européen des brevets

(11) Publication number: **0 197 711**
A2

# EUROPEAN PATENT APPLICATION

(21) Application number: 86302259.6

(22) Date of filing: 26.03.86

(51) Int. Cl.⁴: **A 61 F 5/37**

(30) Priority: 04.04.85 GB 8508946

(43) Date of publication of application:
15.10.86 Bulletin 86/42

(84) Designated Contracting States:
AT BE CH DE FR GB IT LI LU NL SE

(71) Applicant: Pritchard, Barbara Kathleen
Rowan Cottages Rest Home Sulhamstead Road
Burghfield Village Reading RG3 3SB Berkshire(GB)

(72) Inventor: Pritchard, Barbara Kathleen
Rowan Cottages Rest Home Sulhamstead Road
Burghfield Village Reading RG3 3SB Berkshire(GB)

(74) Representative: Singleton, Jeffrey et al,
ERIC POTTER & CLARKSON 27 South Street
Reading Berkshire, RG1 4QU(GB)

(54) Nursing aid.

(57) A Nursing aid comprises a garment having two armholes (4) and a neck opening (6), a front (2), and a back (3), and the width of at least the front of the garment being such that the sides can be tucked beneath the patient. The front (2) of the garment, in use, can serve either as an overlay with respect to clothes covering the patient to prevent the patient gaining direct access to his or her torso, or as a means of limiting patient arm movement by wrapping the front of the garment around the patient's arms and then tucking the sides of the garment beneath the patient to secure in position.

Fig. 1

## NURSING AID

This invention relates to a nursing aid and more particularly to a device for limiting the arm movement of a patient.

It is necessary with some patients to limit arm movement and/or accessibility to certain parts of the body and this is especially so with incontinent patients having senile dementia or being mentally confused and the invention will be discussed with particular regard to such patients but it should be noted that it has a broader application.

Incontinent and doubly incontinent patients with senile dementia tend to want to dislodge their catheter or other relieving device and furthermore tend to want to handle any excreta which is both medically and socially undesirable. Also, such patients do not always take kindly to being cleaned or having their catheter or other device checked or changed and as a result, try to interfere with whatever nursing operation is being effected.

The two main known ways of restraining a patient to avoid the meddling and interference discussed above is by physically restraining the patient or by sedation, neither of which is ideal in that they can be stressful both to the patient and to the patient's visitors and nursing staff. Accordingly, the main objective of the present invention is to provide a nursing aid which effectively limits arm movement in a patient-friendly manner.

Accordingly to the present invention there is provided a nursing aid comprising a garment having two armholes and a neck opening, a front, and a back, and the width of at least the front of the garment being such that the sides can be tucked beneath the patient, the front of the garment, in use, serving either as an overlay with respect to clothes covering the patient to prevent the patient gaining direct access to his or her torso, or as a cover to limit patient arm movement by wrapping the front of the garment around the patient's arms and then tucking the sides of the garment beneath the patient to secure in position.

Normally, the garment is used with a bed-ridden patient but can be used in other circumstances, for example in post-operative care. It will be appreciated that when the patient is in bed, the sides of the garment can and must be firmly tucked beneath the mattress to hold the front of the garment securely in position either in the overlay mode or the restraining mode. In the overlay mode, the patient has complete freedom of arm movement and can be placed to sit or lie down without encumbrance. However, the garment does not allow any direct access by the patient to his or her torso so as to prevent any meddling with catheters, excreta, etc. In the limiting mode, the front of the garment is wrapped around the patient's arms and secured by tucking in the sides, whereby the patient is unable to interfere with any nursing operation being carried out but this is achieved in a very unstressful manner.

Preferably, the front of the garment is longer than the back and in this case the garment may be constructed simply by folding an elongate rectangular piece of material lengthwise but offset from the centre so as to provide the longer front portion and then forming two armholes and a neck opening. Conveniently, the armholes are in the form of elongated slits extending along the fold line, and the neck opening is in the form of a T-shaped slit with the cross piece of the T also extending along the fold line. The armholes and neck opening are preferably reinforced by edge binding but otherwise the garment can be of the material from which normal bed sheets are made and may be of the same or a contrasting colour to that of the patients' bed linen.

However, for violent or strong patients, the garment may be made from a stronger material such as calico.

A nursing aid garment in accordance with the present invention will now be described in greater detail, by way of example, with reference to the accompanying drawings, in which:-

Figure 1 is a perspective view from the front of the garment,

Figure 2 shows the garment of Figure 1 in use in an overlay mode,

Figure 3 shows the garment being changed from the overlay mode of Figure 2 to a limiting mode, and

Figure 4 shows the garment in use in the limiting mode.

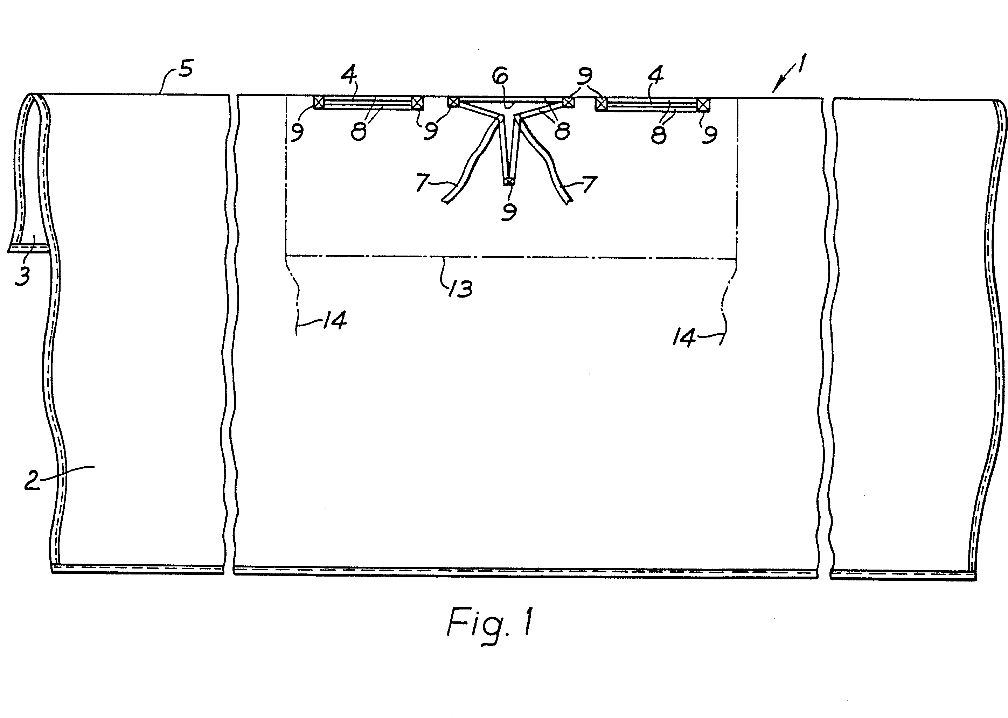

Referring first to Figure 1, the nursing aid garment is indicated at 1 and comprises a rectangular sheet of material which can in fact be a normal single bed size bed sheet measuring 70 inches by 100 inches (180cms x 255cms). The sheet is folded lengthwise but offset from the centre so as to provide a front 2 to the garment and a back 3, the fold preferably being positioned such that the front is twice as long as the back. Two armholes 4 in the form of elongated slits extending along the fold line 5 are provided, together with a T-shaped neck opening 6 of which the cross piece also extends along the fold line. The neck opening 6 is provided with tie ribbons 7 and has bound edges 8, as do the armholes 4. The binding at the corners or ends of the armholes 4 and neck opening 6 is preferably boxed at 9 for extra strength.

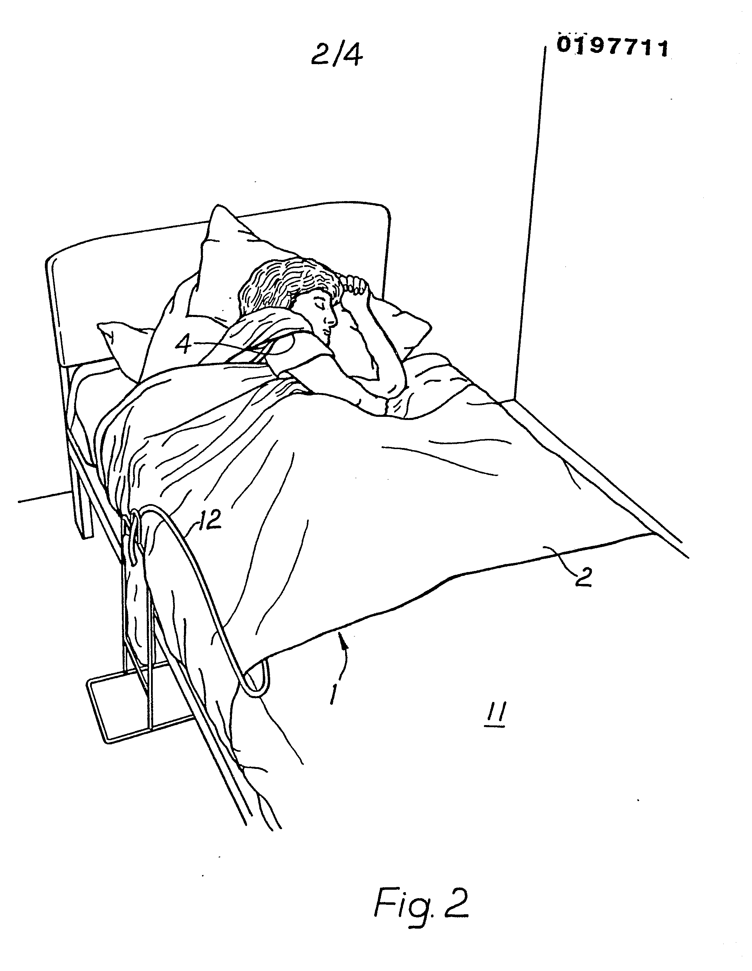

In use, the patient's arms and head are placed through the armholes 4 and neck opening 6, respectively, the garment being in addition to the patient's normal bed attire. In the mode of use illustrated in Figure 2, the front 2 of the garment is used as an overlay with respect to the normal bed linen 11 and as such is pulled down over the bed linen and the sides of the garment tucked beneath the mattress, the relatively large width of the garment providing large tuck-in side pieces to give a very secure fastening of the garment in position. The patient has completely free arm movement in this mode of use and can adopt a sleeping position (as shown in Figure 2) or a sitting position without any encumbrance. In spite of this freedom of movement, the nurse still has some control over the patient's hands and arms.

However, the patient is prevented from gaining direct access to his or her torso and thus meddling with the catheter 12 and tubing with which he or she is fitted. To gain access to his or her torso, the patient would first have to pull up the front 2 of the garment, which in itself is difficult the length of the front portion is beyond the patient's normal reach and in view of the considerable tuck-in of the sides, and then enter between the normal bed sheets. Such an involved and strenuous exercise is not normally undertaken by a patient with senile dementia for whom the nursing aid of the present invention is primarily designed.

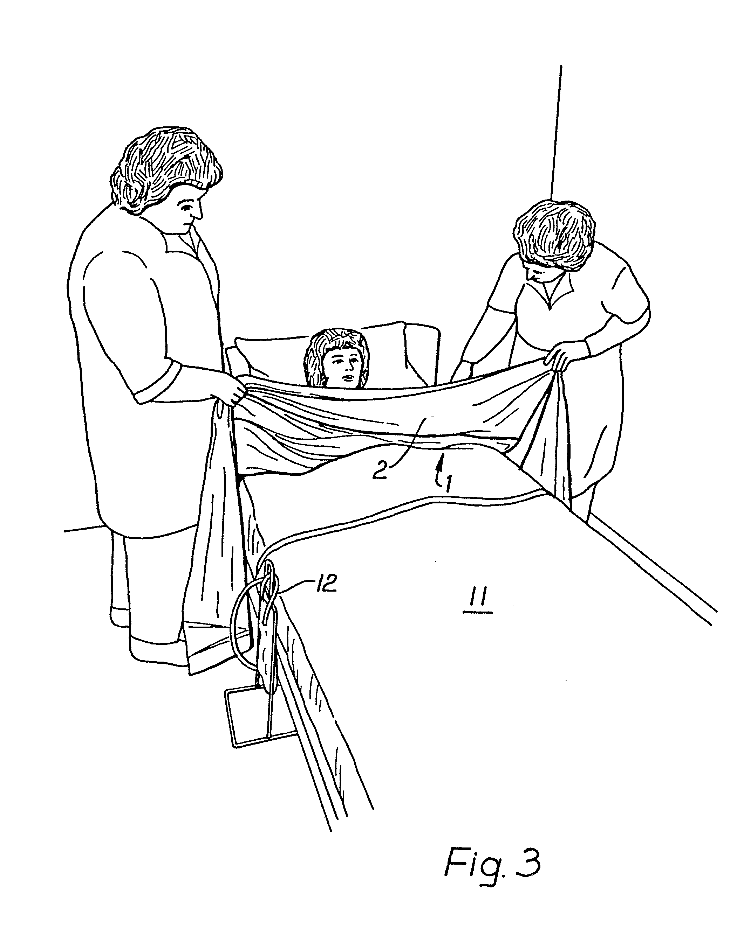

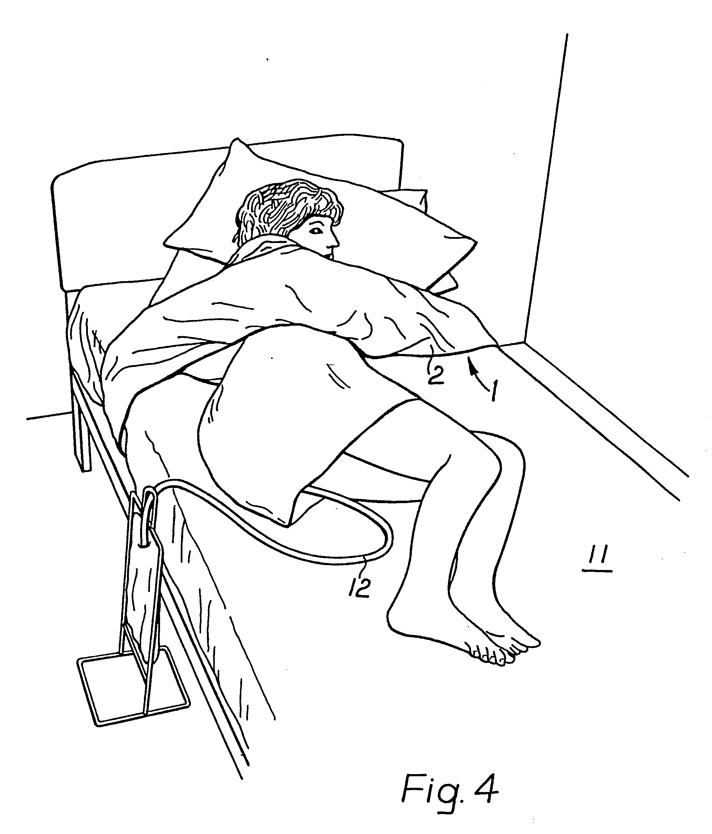

When the catheter 12 is to be checked or changed, or the patient is to be cleaned or otherwise attended to, the garment is changed from the overlay mode of Figure 2 to the limiting mode of Figure 4, part of the changeover procedure being illustrated in Figure 3. The procedure is first to release the front 2 of the garment by untucking the side portions, then turning down the bed clothes and placing the patient in the required position, such as on his or her side, then wrapping the front 2 around the patient's arms and finally re-tucking the side portions, whereby the patient's arms are held very securely but in a painless and unstressful manner whilst the necessary nursing is carried out without interference and thus quickly and efficiently which is to the benefit of both patient and nursing staff.

The side portions of the garment are re-tucked at least generally in line with the patient's shoulders if not further towards the head to keep even the wrapped arms well away from the lower part of the body. Once the nursing has been effected, the garment can be returned to the overlay mode generally by reversing the above procedure. It should be noted that whilst changing from one mode to another the front 2 of the garment can be used as a very effective barrier or screen to prevent not only physical interference by the patient but also visibility of the torso which in some circumstances patients might find distressing, simply by the nursing staff holding the front taut and generally vertical, bearing in mind that normally two members of staff will carry out the changeover procedure. With reasonably operative patients, it may be sufficient to fold the lower part of the front 2 of the garment back over the patient's arms then re-tuck the side portions without needing actually to wrap the arms in the front portion.

It should be noted that the reason for preferring to have the back 3 shorter than the front 2 (and in fact preferably extending only approximately to the base of the spine) is for patient comfort in that to have a longer length of material at the back of the patient would give rise to problems in either tucking the back beneath the patient or folding it up. In either event, the back 3 would tend to ruck up and with any significant length of material involved, and this would cause patient discomfort.

The armholes 4 may be located in any desired position relative to the neck opening 6 but the position shown in Figure 1, i.e. along the fold line 5, has been found to be particularly effective in that the natural forward extension of the patient's arms tends to pull the garment close around the shoulders, thereby very effectively minimising the risk of the patient taking one or both arms back through the armholes 4. It will be appreciated that the size of the armholes 4 and neck opening 6 can be chosen to suit the size of the patient, i.e. the garment can be made for a child, an average sized adult and an above-average sized adult, for example.

Furthermore, the shape and/or orientation of the armholes 4 and neck opening 6 can be varied as required and the ties 7 may be dispensed with. Also, although it is not preferred, the back 3 of the garment may be reduced in width, to effect a saving in material, as indicated by the broken line 13 in Figure 1. In this case, it is preferable to provide ties 14 at the corners of the back 3 which tie around the patient's waist.

It will be seen that the present invention provides a very simple but highly effective nursing aid which affords a very significant advance in the art of nursing especially in the context of the present-day philosophy of total patient care, in which close attention is paid to the psychological well-being, as well as the physical well-being, of the patient.

The garment has an aesthetic appearance and may be colour matched or contrasted with the bed linen so that both the patient and visitors are not in any way disturbed by its presence in the overlay mode. It will be appreciated that the garment serves to prevent patients meddling with any part of the torso so that it can protect not only catheters, as illustrated, but intravenous (I.V.) tubes, dressings, canulae, needles, syringe drivers or other devices which are located other than on the head or arms. Also, when used in the limiting mode, the patient's arms and top portion of the torso are kept warm.

The garment is readily laundered (although it could be made disposable) which is a very important feature and is a very real nursing aid in geriatric wards, psycho-geriatric units, homes for the incurably sick and hospices, for example, as well as in post operative care. Furthermore, the extensive tuck-in portions of the garment result in the patient being very positively retained in bed to the extent that cot sides can in many instances be dispensed with.

Another important benefit resulting from the present invention is that drugs in some cases need not be administered, or at least to a lesser extent, purely for purposes of sedation to prevent the interference and meddling discussed above. This is very much to the benefit of the patient as well as bringing about a significant saving in nursing costs.

## Claims

1. A nursing aid characterised in that it comprises a garment having two armholes (4) and a neck opening (6), a front (2), and a back (3), and the width of at least the front of the garment being such that the sides can be tucked beneath the patient, the front of the garment, in use, serving either as an overlay with respect to clothes covering the patient to prevent the patient gaining direct access to his or her torso, or as a cover to limit patient arm movement by wrapping the front of the garment around the patient's arms and then tucking the sides of the garment beneath the patient to secure in position.

2. A nursing aid according to claim 1, characterised in that the widths of the front (2) and back (3) are equal.

3. A nursing aid according to claim 1, characterised in that the width of the back (3) is less than that of the front (2) and ties (14) are provided on the back which are co-operable with respective ties (15) on the front to retain the back in position.

4. A nursing aid according to any of the preceding claims, characterised in that the front (2) is longer than the back (3).

5. A nursing aid according to any of the preceding claims, characterised in that the garment is formed from an elongate rectangular piece of material folded lengthwise offset from the centre to form the front (2) and back (3), with the armholes (4) formed by elongated slits extending generally along the fold line (5).

6. A nursing aid according to claim 5, characterised in that the neck opening (6) is generally T-shaped with the cross piece (8) of the T also extending generally along the fold line (5).

7. A nursing aid according to any of the preceding claims, characterised in that the edges of the armholes (4) and the neck opening (6) are reinforced by edge binding.

8.    A method of nursing a patient characterised in that it comprises the steps of fitting the patient with a garment having two armholes (4), a neck opening (6), a front (2) and a back (3), and in one mode of use using the front as an overlay with respect to clothes covering the patient to prevent the patient gaining direct access to his or her torso, and in an alternative mode of use limiting patient arm movement by wrapping the front of the garment around the patient's arms and then tucking the sides of the garment beneath the patient to secure in position.

0197711

Fig. 1

0197711

Fig. 2

Fig. 3

0197711

Fig. 4